# EUROPEAN PATENT APPLICATION

(11) **EP 4 092 126 A1**
(43) Date of publication of application: **23.11.2022**
(21) Application number: 22164459.4
(22) Date of filing: 25.03.2022
(51) Int. Cl.: C12P 7/24, C12N 15/52, C12N 9/02

(54) **A METHOD OF ENZYMATIC REDUCTION OF THE OXIDIZED NICOTINAMIDE ADENINE DINUCLEOTIDE AND CARBOXYLIC ACIDS**

(30) Priority: 29.03.2021 PL 43744521; 29.03.2021 PL 43744921
(71) Applicant: INSTYTUT KATALIZY I FIZYKOCHEMII POWIERZCHNI IM.JERZEGO HABERA POLSKIEJ AKADEMII NAUK, 30-239 Kraków (PL); PHILIPPS-UNIVERSITÄT MARBURG, 35037 Marburg (DE)
(72) Inventor: Winiarska, Agnieszka, 30-189 Kraków (PL); Szaleniec, Maciej, 31-201 Kraków (PL); Heider, Johann, D-35041 Marburg (DE); Hege, Dominik, D-35039 Marburg (DE); Arndt, Fabian, D-42329 Wuppertal (DE); Wojtkiewicz, Agnieszka, 30-087 Kraków (PL)
(74) Representative: Godlewski, Piotr

(57) **Abstract**

The present invention relates to a method for the enzymatic reduction of the oxidized form of nicotine adenine dinucleotide to the reduced form of nicotine adenine dinucleotide and biocatalytic method reduction of the carboxylic acids to aromatic and aliphatic aldehydes using hydrogen as reducing agent. The regioselective reduction of the substrates is carried out in an aqueous environment under a hydrogen or hydrogen containing atmosphere using enzyme preparation with tungsten aldehyde oxidoreductase (AOR) activity with an amino acid sequence of at least 70% identity to the sequence deposited in the Universal Protein database Resource Uniprot under the following gene names for subunits AorA ebA5004, AorB ebA5005 and AorC ebA5007, derived from the bacterium *Aromatoleum aromaticum* EbN1, or from the genetically modified bacterium *A. aromaticum* strain SR7 *Δpdh,* or from the recombinant overexpression system in *Aromatoleum evansii* containing a plasmid with genes coding at least the AOR enzyme (i.e. aorA, aorB, aorC genes) and potentially the gene encoding chaperone proteins (aorD and aorE).

## Description

The present invention relates to a method for the enzymatic reduction of the oxidized form of nicotine adenine dinucleotide to the reduced form of nicotine adenine dinucleotide and biocatalytic method reduction of the carboxylic acids to aromatic and aliphatic aldehydes using hydrogen as reducing agent.

Tungsten aldehyde oxidoreductase (AOR) enzymes fall into two separable groups: (I) AOR derived from archaea (such as *e.g. Pyrococcus furiosus*, *Thermococcus strain* ES-1) and (II) AOR enzymes derived from bacteria (such as *e.g. A. aromaticum*, *Thermoanaerobacter* sp. strain X 514).

The AOR enzyme from *Pyrococcus furiosus* was first described in J. Biol. Chem., 1991, 266, 14208-14216 where it is explained that it is an active form of a red tungsten protein (RTP), containing iron-sulfur clusters and previously described by them in J. Biol. Chem. 1990, 265, 11508-11516, but RTP was isolated under aerobic conditions. Isolation under aerobic conditions made it impossible to test the enzyme activity due to its quick deactivation - after 5 minutes of contact with air, only 20% of the activity remain. The temperature optimum of the AOR enzyme from *P*. *furiosus* in the oxidation of aldehydes to acids was determined to be 90°C and ferredoxin and methyl viologen were identified as electron acceptors. The activity of the enzyme was observed for crotonaldehyde, acetic, formic, butyric and glycerol aldehydes.

The AOR enzyme from *Thermococcus* strain ES-1 is described in J. Bacteriol. 1995, 177, 16, 4757-4764, as a biocatalyst for the oxidation of aldehydes, both aromatic (phenylacetic, benzyl, salicylic) and aliphatic (from formic to valeric, also multi-substituted). The temperature optimum for the oxidation reaction was found to be 85°C, indicating the poor thermal stability of the enzyme (50% loss of activity after incubation for 1 min at 70°C). Electron acceptors in this reaction may be benzyl viologen, methyl viologen and ferredoxin. The paper also shows the possibility of reducing acetic acid to acetaldehyde with the use of reduced methyl viologen with a kinetic rate constant (k_{cat}) of 1.6 turnovers of the catalyzed reaction per second. The AOR enzyme from *Thermococcus* strain ES-1 is very sensitive to oxygen, loses 50% of its activity after contact with air after 1 minute and does not regain activity after oxygen removal.

The bacterium *Azoarcus sp.* EbN1 (*Aromaticum aromatoleum*) was isolated by R. Rabus and F. Widdel from a denitrifying culture enriched with ethylbenzene as the sole carbon source. The method of isolating and culturing *Aromaticum aromatoleum* (EbN1, formerly genus *Azoarcus*) was disclosed in Arch. Microbiol., 1995, 163, 96-103.

When EbN1 bacteria are cultivated under anaerobic conditions on a medium containing phenylalanine as a carbon source, an intermediate metabolite, phenylacetylaldehyde, is formed. Phenylacetylaldehyde is catalytically oxidized to phenylacetylic acid either by phenylacetaldehyde dehydrogenase or low quantities of tungsten aldehyde oxidoreductase (AOR).

The report in J. Bacteriol. 2017, 199, e00383-00317 discloses the cultivation conditions of *A. aromaticum* strain SR7Δpdh (*i.e.* streptomycin-resistant *A. aromaticum* with a deletion of the gene coding for phenylacetaldehyde dehydrogenase). This *A. aromaticum* strain efficiently expresses the gene for AOR in high quantities that allow enzyme isolation by chromatographic methods.

Reduced nicotinamide adenine dinucleotide (NADH) is a cofactor of numerous enzymes belonging to the class of oxidoreductases. It is necessary for several important biotechnological processes that run under mild conditions with high regioselectivity and often with high enantioselectivity. Examples of such processes are enantioselective reduction of ketones to secondary alcohols, catalyzed by alcohol dehydrogenases, regioselective reduction of aldehydes to alcohols by aldehyde dehydrogenases, enantioselective reduction of double bonds by ene-reductases or oxidation reactions of organic compounds catalysed by cytochrome P450-type enzymes. Because of the high cost of NADH (approx. $ 2,600/mol) the industrial application of these enzymes is dependent on the chemically efficient *in situ* reconstitution of the NADH cofactor in all these cases.

The following methods of reduction of NAD⁺ to NADH are known from the literature: i) enzymatic methods using hydrogenases or dehydrogenases, ii) photocatalytic methods, ii) electrocatalytic methods, iv) homogeneous and heterogeneous catalytic methods and v) chemical reduction methods. Especially important from the modern chemistry point of view are the enzymatic methods. So far, only enzymatic NADH regeneration systems have been used in industrial practice due to their high selectivity, catalytic activity and their possible applicability in crude extract or whole cell biotransformation systems.

In Front. Microbiol., 2019, 10, 71 the quaternary structure of AOR, the optimal pH of the aldehyde oxidation reaction (8.0) and the optimal reaction temperature (40°C) was described. Moreover, the enzyme's ability to reduce nicotinamide adenine dinucleotide (NAD⁺) to 1,4-NADH with aldehydes was revealed. In particular, the observed kinetic parameters were determined for the reaction in which the enzyme oxidized 1 mM benzaldehyde and was re-oxidized with NAD⁺. The process was carried out at pH 8.0 at 28°C under anaerobic conditions. Under these conditions, the maximum observed reaction rate, Vₘₐₓ, was 12.5 mM min⁻¹ mg⁻¹ and the Kₘ versus NAD⁺ was 63 µM.

The review FEBS J. 2013, 280: 13, 3058-68 describes the use of [FeFe]- and [NiFe]-hydrogenases catalyzing the reduction of NAD (P)⁺ with the use of hydrogen. For example, [NiFe] dehydrogenase from *Ralstonia eutropha*, exhibiting resistance to the reversible inhibition by O₂, has been used to regenerate NADH. The specific activity of the enzyme was 137 U mg⁻¹ at pH 8.0, 30°C and in the absence of sodium ions. The enzyme showed a high affinity to hydrogen (Kₘ = 11-37 µM 1.4-5% H₂) and moderate affinity to NAD⁺ (Kₘ 560 µM). Under hyperbaric conditions, the enzyme showed inhibition by H₂ (Kᵢ = 1.46 mM).

In Biotechnol. Lett. 1989, 4, 673-678 the stabilization of hydrogenase by immobilization on porous glass was revealed, which allowed a 15-fold extension of the enzyme activity under process conditions, *i.e.* from 10 to 150 h. However, the immobilization efficiency was only 23% and did not improve the temperature stability of the catalyst.

An example of the use of formate dehydrogenase (FDH) to regenerate NADH can be found in Ka-Yiu San, Susan J. Berrios-Rivera and George N. Bennett US7709261B2. FDH catalyses the oxidation of formic acid to carbon dioxide, which is released from the solution when it exceeds its solubility. However, this system is characterized by low specific activity (~10 U mg⁻¹) and exhibits no resistance to organic solvents. Moreover, the reaction results in acidification of the aqueous solution by carbonic acid.

Another example of an NADH regeneration system is described in patent application WO 2006/074194 A2. The invention uses phosphine dehydrogenase (PTDH) from *Pseudomonas stutzeri*, which catalyzes the oxidation of phosphate (III) ions to phosphate (V) ions while reducing NAD⁺ to NADH. The application describes enzyme mutants with increased stability and improved kinetic parameters relative to the native form. The process can be carried out in the temperature range 40-50°C, at a pH of about 7.5. The enzyme shows a high affinity to NAD⁺ (Kₘ 53 µM) and resistance to the presence of arsenate, sulphate, nitrate and NaCl ions.

Furthermore, in App. Microbiol. Biotechnol. 2015, 99, 5055-5069 another solution was presented for (S)-1-phenylethanol dehydrogenase (S-PEDH) from *A. aromaticum,* which catalyzes the reduction of ketones to secondary alcohols. NADH consumed in the reaction was regenerated by adding high concentrations of isopropanol as sacrificial co-substrate, which was oxidized in a reverse reaction by the enzyme to acetone, continuously recycling NADH. The enzyme was introduced into the reactor both in isolated form and as in a whole cell system of recombinant *E. coli* cells. S-PEDH showed exceptionally high resistance to organic reagents, which allowed the process to be carried out in 60% isopropanol. Furthermore, US patent application US 2010 0143991 disclosed that this enzyme can regenerate NADH also using butan-2-ol or pentan-2-ol.

The report in Energy Environ. Sci. 2013, 6, 1486-1493 discloses a photocatalytic system that enables NADH regeneration. The regeneration system consisted of a photosensitizer, *i.e.* a carbon nitride on diatomaceous earth support, a rhodium complex mediator ([Rh(cp(CH₃)₅)(bpy)H₂O]²⁺) and a reducing donor, triethanolamine. Regeneration was effective in the pH range of 7-10, optimally 8.0. The regeneration of 1 mM NAD⁺ in the presence of 0.25 mM mediator and 15% (w/v) reducing agent required 2 hours of irradiation with light at 420 nm. However, the described process cannot be used in a bacterial cell and also leads to contamination of the reaction mixture with transition metal compounds. Moreover, in J. Mol. Catal. B: Enzymatic 2010, 63, 149-156 it was shown that the mentioned mediator has a denaturing effect on the model enzyme (alcohol dehydrogenase from *Thermus* sp. ATN1) leading to its inactivation. It is possible to carry out the regeneration process without the mediator, but then the system efficiency is reduced to 50%.

In Top. Catal. 2014, 57, 321-331 numerous methods of NADH regeneration were revealed that based on homogeneous catalytic systems. For example, in a method described in more detail in the original work of J. Am. Chem. Soc. 2012, 134, 367-374, the iridium complex ([Cp*Ir(4-(1H-pirazol-1-yl)benzylic acid)H₂O]SO₄) was used as a catalyst. This system used hydrogen as NAD⁺ reducing agent and operated at pH 6-8 and room temperature and under anaerobic conditions at atmospheric pressure. However, the system was able to regenerate NADH for approximately 9 turnovers (at system reaction rate of approximately 1 mM/h at NAD⁺ concentration of 30 µM). Moreover, under acidic conditions (pH 4-6, favoring the reduction of organic compounds) the complex preferentially catalyzed the reverse reaction, *i.e.* the oxidation of NADH to NAD⁺. The observed reaction rate (TOF) also decreased with increasing NAD⁺ concentration due to the substrate inhibition of the catalyst which prevented binding of the hydrogen. As a result, the process was effective only below 1 mM of NAD⁺.

In Chem. 2017, 2, 621-654 several examples of the use of metallic heterogeneous catalysts were disclosed, where the active phase was Pt, Pd, Rh, Ru, Ni, Au, or Ag, supported on Al₂O₃ or carbon materials. In particular, in ACS Catal. 2016, 6, 1880-1886, the catalyst Pt (1%)@Al₂O₃ was disclosed, which was able to regenerate NADH in a wide pH range (4-9.9) at the H₂ pressure range of 1-9 atm under the anaerobic atmosphere. The activity of the system decreased with a decrease in temperature (from 60°C to 25-37°C by approx. 50% ), a decrease in pH (from optimal at 9.9 to 7.0 by 33%) and a decrease in hydrogen pressure from 9 atm to 1 atm (by 90%).

In the literature there are also examples of NADH regeneration with chemical methods that utilize reducing agents. An example of such a procedure is described in Biochim. Biophys. Acta. 1979, 9; 571, 171-4, where sodium cyanoborohydride (NaBH₃CN) was used to reduce NAD⁺. The reduction process was effective at pH 5-8 with a large excess of reductant (10-20 M versus 1-10 mM NAD⁺). Under such conditions, the process was slow (0.2 µM/s), but the correct 1,4-NADH isomer was formed. This system is not suitable for use in the presence of enzymes due to the very high concentration of the reductant.

Aromatic and aliphatic aldehydes constitutes an important group of compounds indicating many important roles in chemistry and medicine.

As reported Int. J. Toxicol., 2006, 25 (Suppl. 1):11-27 benzaldehyde is used as a component of a fragrance, flavoring or denaturating agent and its derivatives have been tested as drug action cancerostatic.

According to the patent application US20180289636A1, 4-anisaldehyde (CAS 123-11-5) has the potential to be used in the treatment of dysphagia (dysphagia), a therapy aimed at reducing body weight and reducing blood glucose levels.

As reported in European Patent EP2437737B1 trans-cinnamaldehyde (CAS 14371-10-9) may be a component of mixtures for therapeutic or preventive action against bacterial infections. The described mixtures have an antimicrobial effect on both gram-positive and gram-negative bacteria and can be effective against such non-resistant and/or resistant or even multi-resistant bacteria.

Trans-cinnamaldehyde has been approved by the Food and Drug Administration (FDA) for use in allergy patch tests, which are indicated for use as an aid in the diagnosis of allergic contact dermatitis (ACD) (Drugbank accesion number DB14184).

According to the patent US2721879, a range of aliphatic aldehydes from formaldehyde to heneicosal can be advantageously used as a modifier preventing gelling and crosslinking of the product in copolymerization for unsaturated carboxylic acids.

An example of enzymatic acid reduction by carboxylic acid reductase (CAR) can be found in Chemcatchem, 2019, 11, 4171-4181. This process uses CAR to reduce the carboxylic acids activated by adenosine 5'-triphosphate (ATP). The process also requires the presence of a reducing agent in the form of the reduced nicotinamide adenine dinucleotide phosphate (NADPH), which also implies the need to use a regeneration system for NADPH, for example employing glucose dehydrogenase, where the electron donor was glucose. The process used recombinant CARs from *Trametes versicolor* and *Dichomitus squalens* expressed in *E. coli* as a whole-cell catalyst at an optimal temperature of 25-35°C and at pH in the range of 7-8.The enzyme was active in the reduction of several aromatic acids (*i.e.*, benzoic acid, piperonylic acid, 3,4-dihydroxyphenylacetic acid) and medium-chain C5-C10 fatty acids. However, only for 3,4-dihydroxyphenylacetic acid a practical example of reduction process was revealed yielding 3-hydroxytyrosol *(i.e.* alcohol) which was reduced with an endogenous pool of reducing agents and alcohol reductase from *E. coli* BL21. The process was conducted with whole-cells from a high cell density culture (OD 50) and yielded 49.4 mM of the product from 60 mM of the substrate during 21 h of reaction.

Enzymatic reduction of benzoate to benzaldehyde by AOR from *A. aromaticum* was recently reported in Front. Microbiol., 2019, 10, 71 with Ti(III) citrate as reductant and the general acid-reducing capability of AOR-type enzymes has previously been revealed for enzymes from anaerobic bacteria and archaea, which are only distantly related to AOR of *A. aromatoleum* (48-52% identity of the catalytic AorB subunits). The enzyme from *Moorella* *thermoacetica* reduced various acids in an electrochemical setup with tetramethylviologen as mediator, which was described in Eur. J. Biochem, 1989, 184, 89-96 and Arch. Microbiol., 1995, 164,110-118, whereas archaeal AOR reduced acetate with chemically reduced methylviologen according to paper J. Bacteriol., 1995, 177, 16, 4757-4764 . However, none of the previous experiments used molecular hydrogen as reductant for acid reduction.

The role of enzymes from the group of aldehyde oxidoreductases (AOR) in the biocatalytic reduction of carboxylic acids to alcohols in the cascade with alcohol dehydrogenases and their industrial potential has been described by L. Nissen, M. Basen in J. Biotech. 2019, 306, 105-107. For example, cells of *Thermoanaerobacter sp.* strain X514 with a culture density corresponding to a protein concentration of 1-2 mg mL⁻¹ protein produced up to 43 mM isobutanol from isobutyrate, with glucose as electron donor, with a recovery of carbon atoms above 85%.

The article Front. Microbiol., 2019, 10, 71 describes the quaternary structure (AorB AorA AorC)₂ of the AOR enzyme, the optimal pH of the aldehyde oxidation reaction (8.0) and the optimal reaction temperature (40°C) and the substrate specificity for a number of aromatic aldehydes (benzaldehyde, p-hydroxybenzaldehyde, 2-aminobenzaldehyde) of alkyl aldehydes (phenylacetaldehyde) and aliphatic aldehydes (formaldehyde, acetic aldehyde, crotonic aldehyde, gliceral, glutaral), using benzyl viologen or nicotinamide adenine dinucleotide (NAD⁺ ) as electron acceptors. This article also shows that the catalyzed oxidation of aldehydes to acids can be a reversible reaction, provided that strong chemical reductants such as titanium (III) citrate and low pH are used. This process was demonstrated on the example of the reduction of benzoic acid to benzaldehyde, but it was very slow and the products were formed in trace amounts that prevented their isolation.

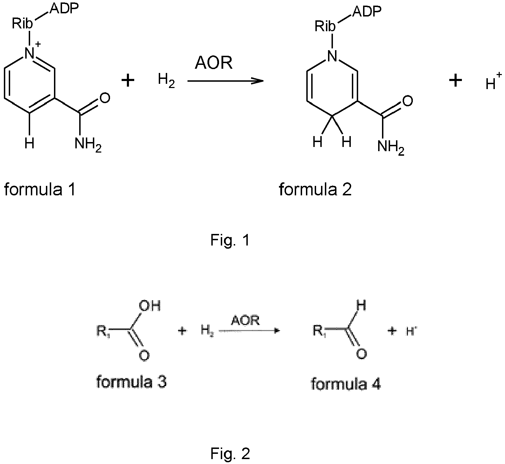

The invention aims to provide a simple and effective method for the enzymatic reduction of NAD⁺ (formula 1) to the reduced nicotine adenine dinucleotide (1,4-NADH, formula 2) using hydrogen gas as an ecological and clean reducing agent (figure 1), whereas Rib is Ribose and ADP is adenosine 5'-diphosphate. The object of the invention is also to provide a biocatalytic method for the direct hydrogen-mediated reduction of carboxylic acids (formula 3) to aldehydes (formula 4) by AOR (figure 2), whereas R₁ is an aliphatic chain with a length of one to seven carbon atoms, or an aromatic group, or a furan group, or a thiofuran group, or a 3-pyrimidine group, or a benzyl group, or a propionic group, or ethylcarboxyl group, or a 2-ethylphenyl group or an ethyl-1,2-diol group, or 2-propene group, or C₆H₅ group, or F-C₆H₄ group, or Cl-C₆H₄ group, or Br-C₆H₄ group, or NH₂-C₆H₄ group, or OH-C₆H₄ group, or OMe-C₆H₄ group, or 4-OH-3-OMe-C₆H₃ group, using hydrogen as an ecological and pure reducing agent.

It was found that the enzyme preparation with tungsten aldehyde oxidoreductase (AOR) activity derived from the bacterium *Aromatoleum aromaticum* EbN1 is is not only reduced by aldehydes, but also by molecular hydrogen. In turn, the hydrogen- reduced enzyme reduces NAD⁺ of formula 1 to 1,4-NADH of formula 2, under a nitrogen atmosphere containing 0.1 to 100% (v/v) hydrogen and at a pressure range from 1-2 atm., after incubation for a period of up to 48 hours in an aqueous buffer solution of pH 5-9 (Fig. 1).

The reduced enzyme is able also to reduce carboxylic acids of formula 3 to aldehydes of formula 4 in the atmosphere composed of a mixture of inert gas (e.g. nitrogen, helium, neon, argon, krypton, xenon, methane, carbon dioxide) and hydrogen with a concentration ranging from 0.1 to 100% (v/v), in the pressure range from 1-5 atm., up to 48 hours in aqueous phosphate buffer solution, hydrochloride, tris(hydroxymethyl)aminomethane (TRIS), 2-(N-morpholino)ethanesulfonic acid (MES) of pH 4.0-7.5 (Fig. 2).

According to the invention, the process of enzymatic reduction is characterized by the fact that the regioselective reduction is conducted under an anaerobic atmosphere containing hydrogen, by an enzyme preparation with tungsten aldehyde oxidoreductase (AOR) activity with an amino acid sequence of at least 70% identity to the sequence deposited in the Universal Protein database Resource Uniprot under the following gene names for subunits AorA ebA5004, AorB ebA5005 and AorC ebA5007, derived from the bacterium *Aromatoleum aromaticum* EbN1, or from the genetically modified bacterium *A. aromaticum* strain SR7 *Δpdh*, or from the recombinant overexpression system in *Aromatoleum evansii* containing a plasmid with genes coding at least the AOR enzyme (*i.e.* aorA, aorB, aorC genes) and potentially the gene encoding chaperone proteins (aorD and aorE), whereby the regioselective reduction of the substrate of formula 1 to product of formula 2 is carried out in an aqueous environment, at a temperature of 5 to 40°C, in the reaction mixture, which contains:
- a buffer providing a pH in the range of 4.0-9.0,
- the carboxylic acid solution at a concentration of 0.1 mM to 100 mM,
the enzyme preparation maintaining the biocatalytic activity of tungsten aldehyde oxidoreductase for a period up to 48h, which enables the process of regioselective reduction of carboxylic acids has the following course: into the batch reactor under anaerobic atmosphere the buffer, substrate dissolved in water or buffer and enzyme are introduced and then the reaction is initiated by the introduction of the inert gas containing hydrogen or pure hydrogen and the course of the reaction is monitored and product is separated by means of solid phase extraction or liquid-liquid extraction in according with the rules of the art.

Preferably, the regioselective reduction is carried out in an atmosphere consisting of a mixture of hydrogen and inert gas, in which the hydrogen concentration is 0.1-100% (v/v), Preferably, the regioselective reduction is carried out in an atmosphere consisting of a synthetic gas which is a mixture of carbon monoxide, inert gas, hydrogen and trace amounts of oxygen (<0.05% v/v).

Preferably, the AOR catalyzing enzyme present in the enzyme preparation is characterized by a heteromeric structure comprising at least 3 subunits (AorA, AorB and AorC), the amino acid sequences of which have been deposited with Uniprot under the following reference numbers: for AorA subunit ebA5004, AorB ebA5005 and AorC ebA5007.

Preferably, the AOR catalyzing enzyme present in the enzyme preparation has an amino acid sequence with an identity of at least 70% with respect to the amino acid sequences with the ID codes deposited in the Universal Protein Resource (UniProt) database under the following AorA subunit reference numbers ebA5004, AorB ebA5005 and AorC ebA5007.

The AorA ebA5004subunit has following sequence:

MWKSLHIDPAKCTGCLQCEMACSYEHTGVINPSKSRIKVFSFEHEGRKVPYTCTQCTEAWC
LHSCPVDAIRLDLTTGAKMVFEDTCVGCKVCTIACPFGTINYNQDTGKVQKCDLCEGDPACA
KACPTAAITYIDADWTGLARMQAWAAKANTPASAA

The AorB ebA5005 subunit has following sequence:

MGWNRKVLRVNLAEGTCTPEPLNMQWADEYLGSRGLATKYLVSETDPKVDPLSPDNKMIM
ATGPLTGTMASTGGRYTVVTKGPLTGAIACSNSGGFFGAEMKFAGWDMVIFEGRSPTPVYL
FIENERAELRDASYLWGRSCWETEESIRAQHQDPLIRVSSIGRAGENQVMFACIVNDLHRAA
GRSGVGAVMGSKNLKAVAIRGTKGVSGIRDFPGFVRATSEAKKVLAGNPVTSEGLPKFGTQ
VLMNVINEMGALPTRNHRDVQFEDASKISAEAMHEKRPSDGKPQLVTNAACFGCTIACGRIS
AIDKTHFTVKNNPKYWGASGGLEYEAAWALGAANGVGDLEALQYANLLCNEQGMDPISFG
ATVGAAMELYETGVLTKERIGLDAPFGSADALAKLAEMTATGEGFGKEIGLGSKRLCEKYGH
PELSMSVKGQEFPAYDSRGIQGMGLAYATSNRGACHLRGYTVASEVLGVPVKTDPHVIEGK
AELVKAFQDATAVFDSAGICVFTSFAWTLADVQPQIAAACDGDWSMDKLATVGERIWNMER
QFNNAAGLGAQDDNLPPRLTSEPAKSGPAKGMVNRLAEMLPEYYGVRGWTPEGTPTPETL
SRLGLS

The AorC ebA5007subunit has following sequence:

MKHVILGNGPAGVIAAETLRRAAPTDDILLFGSEDAPPYSRMAIPYLLEGNIDESGTWLRKSP
GHFDRLRIHEMRGRAVSLDSERRRILFDDGHFESWDRLLIATGSHPVRPPIPGIDLPEVQTC
WTLEDARAIARFATPGARVLQLGAGFIGCIIMEALAARGVELTVVEMGDRMVPRMMTPTAG
GMIRKWVEDQGVRVVTNAGVSRIDCRASNDAPLDVTLSTGEVVVADLVIVAAGVAPNIAFLE
ATPVHVAKGVLVDDRLQTSVPGIFAAGDVAEAPDLFTGAHLVAAIQPNAADQARVAALNMA
GHEARLKGVLAINVLDTLGLISSSFGQWWGEERERGGAGVEHVDEAAYRYLSLQFKDDVLI
GATSIGLTEHVGALRGLIHGRVRLGEWKERLLHSPLQFVDAYIARSQQPMALVR

Preferably, the regioselective reduction process is carried out at a temperature of 15 do 40°C, more preferably 30 do 40°C.

Preferably, the regioselective reduction process is carried out in an atmosphere consisting of a mixture of hydrogen and inert gas or in pure hydrogen under the pressure of 1-5 atm., more preferably under the pressure of 1-2 atm.

Preferably, during the regioselective reduction of carboxylic acids, the equilibrium of the reaction is shifted in favour of the aldehyde products by selectively removing the aldehyde products from the reaction medium.

Preferably, the selective removal of aldehyde products from the reaction medium is carried out by known liquid-liquid extraction methods.

Preferably, the pH of the carboxylic acids reduction reaction is maintained in the range of 4.0-7.5, preferably 4.5-7.0, more preferably 5.0-7.0.

Preferably, the pH of the reaction medium is controlled by the organic acid substrate. Preferably, the pH of the NAD⁺ reduction reaction is maintained in the range of 5.0-9.0. Preferably,the PH of the reaction medium is controlled by a buffer selected from tris(hydroxymethyl)aminomethane hydrochloride, 2-(*N*-morpholino)ethanesulfonic acid, 2,2-bis(hydroxymethyl)-2,2',2"-nitrilotriethanol hydrochloride, 2-[(2-amino-2-oxoethyl)-(carboxymethyl)amino]acetic acid, phosphate buffer, buffer based on citric acid.

Preferably, the regioselective reduction of NAD⁺ is carried out at the carbon 4 of the nicotinic ring of the nicotinamide adenine dinucleotide.

Preferably, the regioselective reduction of carboxylic acids is monitored by HPLC, or HPLC coupled with MS, or MS/MS.

Preferably, the regioselective reduction of NAD⁺ is monitored by a spectrophotometric method (absorbance measurement at 340 nm).

Preferably, the method according to this invention comprise at least one additional step of recovering and/or purifying the product, whereat the product is either 1,4-NADH of formula 2 itself or a fermentation product produced by a microorganism being supplied with 1,4-NADH of formula 2 produced by the method according to any one of preceding claims 1 to 9, the at least one additional step of recovering and/or purifying comprising at least one recovering or purifying method independently chosen from the list comprising the recovering and/or purifying methods filtration, sedimentation, centrifugation, distillation, liquid-liquid extraction, chromatography, HPLC, adsorption to a solid phase combined with desorption from a solid phase, vacuum-drying, lyophilisation, crystallisation.

In the context of the present invention, the term "enzyme preparation" is understood as a crude enzyme, containing, apart from AOR, other proteins, including enzymes which, due to the nature of the substrate and the reaction mixture, do not exhibit activity and do not modify the substrate in any other way than NAD⁺ reduction or carboxylic acids reduction to aldehydes and hydrogen oxidation.

The term "biocatalytic process" in the context of this invention refers to any process carried out in the presence of AOR enzymatic activity, whether in a purified, crude, cell-free extract or bacterial cell form, provided that they exhibit under reaction conditions only tungsten aldehyde oxidoreductase activity toward reaction substrates.

In the context of this invention, the term "regioselective" is understood as a process that takes place only at the carbon 4 of the nicotinic ring in the nicotinamide adenine dinucleotide or is understood as a reduction process that proceeds exclusively with respect to the carboxylic acid group.

The method according to the invention enables obtaining NADH, respectively recycling NAD⁺ to NADH, which is a valuable cofactor in reduction reactions catalyzed by oxidoreductases, which allows for the synthesis of organic compounds valuable for the pharmaceutical, cosmetic and fine chemistry industries.

This method can be used to obtain aromatic aldehydes, substituted aromatic aldehydes, heterocyclic aldehydes, saturated and unsaturated aliphatic aldehydes, aliphatic aldehydes with hydroxyl groups which are high-value synthons for the pharmaceutical, cosmetic and fine chemistry industries. The method of reducing carboxylic acids to aldehydes according to the invention is regioselective.

The method according to the invention may find application in the chemical industry for the production of various aldehydes by a single enzyme with the aid of hydrogen as cheap and readily available reductant. The generated aldehydes can be applied in chemical and pharmaceutical synthesis (drugs and drug precursors) as well as in the food and perfumery industry (flavours and fragrances), and as a raw material for syntheses and plasticizers in the plastics industry. An additional advantage is the selectivity of the reduction method, i.e. no subsequent reaction to alcohol and no reduction of double bonds or aromatic systems.

The invention is presented in the following figures:
Fig. 1 General scheme of NAD+ (formula 1) reduction to 1,4-NADH (formula 2) catalysed by tungsten aldehyde oxidoreductase from *A. aromaticum* EbN1 using hydrogen as an electron donor.
Fig. 2 General scheme of carboxylic acid (formula 3) reduction to aldehydes (formula 4) catalysed by tungsten aldehyde oxidoreductase from *A. aromaticum* EbN1 using hydrogen as an electron donor.
Fig. 3 Schematic representation of NAD⁺ reduction process with H₂ catalyzed by AOR.
Fig. 4 UV-vis spectra in the 200-400nm range collected during NAD⁺ reduction.
Fig. 5 Change in absorbance over time at 340 nm.
Fig. 6 Schematic representation of the reaction of 4-nitroacetophenone reduction to (R)-1-(4'-nitrophenyl)ethanol catalysed by the R-HPED coupled to the reduction of NAD⁺ with H₂ catalyzed by AOR.
Fig. 7 Schematic representation of the reaction of acetophenone reduction to (R)-1-phenylethanol catalysed by the R-HPED coupled to the reduction of NAD⁺ with H₂ catalyzed by AOR .
Fig. 8 The course of the reaction of acetophenone to (*R*)-1-phenylethanol reaction catalysed by R-HPED with NADH regeneration system of crude extract of recombinant AOR and H₂. The average reaction rate approximated by linear trend was 18 µM/h.
Fig. 9 Change in absorbance with time at 340 nm.
Fig. 10 A reaction scheme for the reduction of benzoic acid to benzaldehyde with hydrogen catalyzed by AOR.
Fig. 11 Change of benzaldehyde concentration during the reaction of reduction of benzoic acid with hydrogen in the presence of AOR with *A. aromaticum* SR7*Δpdh.*
Fig. 12 AOR catalyzed reaction scheme for the reduction of transcinnamic acid to transcinnamaldehyde with hydrogen.
Fig. 13 AOR catalyzed reaction scheme for the reduction of octanoic acid to octanal with hydrogen.
Fig. 14 MS spectrum of the isolated product (upper left corner), a spectrum of the octanal standard from the replib library (lower left corner) and the difference between these spectra (right plot).
Fig. 15 AOR catalyzed reaction scheme for the reduction of 4-hydroxybenzoic acid to 4-hydroxybenzoic aldehyde with hydrogen.
Fig. 16 AOR catalyzed reaction scheme for the reduction of vanillic acid to vanillin with hydrogen.
Fig. 17 A) GC-MS chromatogram of reaction mixture with vanillin (retention time 12.3 min) and vanillic acid (retention time 15.3 min); B) MS spectrum of the vanillin; C) MS spectrum of the vanillin standard from the mainlib NIST library.
Fig. 18 AOR catalyzed reaction scheme for the reduction of nicotinic acid to nicotinic aldehyde with hydrogen.
Fig. 19 MS spectrum A) isolated product, B) spectrum of 3-pyridinkaboxyaldehyde standard from NIST replib library and C) differential spectrum of spectra A and B.

The invention is explained in detail in the following embodiments.

### Example 1. Isolation of enzyme preparation with AOR activity from Aromatoleum aromaticum EbN1 bacteria, strain SR7Δpdh.

The procedure of obtaining a native enzyme preparation with AOR activity consists of two stages: bacterial culture and isolation of the enzyme preparation with AOR activity from the bacterial mass. The cultivation of *Aromatoleum aromaticum* EbN1 strain *SR7Δpdh* was carried out in a minimal carbonate buffered medium (NaHCO₃) containing 150 nM Na₂MoO₄ and 18 nM Na₂WO₄ of the highest purity (99.9 and 99.995%, respectively) with phenylalanine as the carbon source (1 mM) and sodium nitrate (4 mM) as an electron acceptor in an anaerobic environment. Cultures were periodically fed with identical aliquots of substrates (i.e. aliquots of phenylalanine and sodium nitrate giving a final concentration of 1 mM and 4 mM, respectively) and grown at 28°C. Growth was monitored by measuring the optical density observed as absorbance at 578 nm (OD578). Cells were harvested after reaching an optical density in the range of 1-1.5 by centrifuging the culture in a centrifuge at 17,000 g and 4°C for 20 min. The sedimented cells were frozen and stored at -80°C.

Frozen cells were thawed anaerobically in the ratio of wet cell weight to 1: 1 (w:v) buffer using 20 mM 2-bis(2-hydroxyethyl)amino-2-(hydroxymethyl)-1,3-propanediol buffer (Bis-Tris) pH 6.2 containing 0.05 mg/ml DNase I and 10% glycerol. The bacteria were homogenized by ultrasonication (Sonics Vibra-Cell VCX500, intermittent cycle, 5 min, amplitude 40%, energy 150,000 J) or by processing them three times by a French press. The solution was then subjected to ultra-centrifugation (1 h, 100,000 g, 4°C) to separate the membrane fraction and purify by preparative chromatography. For this purpose, a two-step method was used, using deoxygenated buffers (described below) cooled to 16°C. The cell extract was filtered through a 0.45 µm filter before being applied to the column. In the first step, the extract was purified on a DEAE-sepharose Fast Flow preparative column equilibrated with 20 mM Bis-Tris buffer, pH 6.2. The AOR was eluted in a gradient mode with 20 mM Bis-Tris buffer, pH 6.2, 1 M NaCl, resulting in a NaCl concentration of 400 mM in the harvested protein fraction. The active protein fractions were pooled and desalted using a desalting column (e.g. HiPres (26/10) GE-Healthcare) and resuspended in 5 mM MES buffer, pH 6.9, 2 mM CaCl₂. In the second step, protein fractions were separated on a ceramic hydroxyapatite column (CHT-I, 70 ml) equilibrated with 5 mM MES buffer, pH 6.9, 2 mM CaCl₂. The active fraction was eluted with a gradient between 5 mM MES buffer, pH 6.9, 2 mM CaCl₂, and 5 mM MES buffer, 400 mN K₃PO4, pH 6.8 at a phosphate concentration of 15 mM. The collected fractions were concentrated by ultrafiltration on a 30 kDa membrane. This method was used to obtain the enzyme with an efficiency of 70% with respect to the activity in the cell extract and a 26-fold enrichment of the preparation with the AOR enzyme, with the final specific activity of about 7 µmol min-1 mg-1 in the phenylacetaldehyde oxidation activity test with benzyl viologen as an electron acceptor.

### Example 2. Isolation of enzyme preparation with AOR activity from A. aromaticum overexpressed in A. evansii.

This example demonstrates the preparation of the AOR enzyme in recombinant form using the *A. evansii* expression system.

The genes encoding the AOR protein from *A. aromaticum* (gens EB_RS13980-14000) are isolated according to the art by PCR amplification with the primers indicated in Table 1 and ligated to the mobilization vector. The obtained plasmid pASG_105_AOR_full is transformed into a conjugate strain of *Escherichia coli* (e.g. strain S17-1). The recombinant AOR contains an additional fused strep-tag sequence at the N-terminus of the AorA subunit, which does not interfere with its activity and allows fast purification by affinity chromatography.

The forward (fw) and reverse (rv) primer pairs used are:

**Table. 1. Primers used for the amplification of AOR genes by PCR.**

| Seq no | Name | Sequence (5'---> 3') |
|---|---|---|
| 1. | AorA_ebA5004fw | 5’AAGCTCTTCAATGTGGAAATCGCTTCACATTGAC CC3’ |
| 2. | AorE_ebA5010stop3r v | 5'AAGCTCTTCACCCCATTGCTTGCTGCGCTCGTCT G3' |

The plasmid is then transferred from the donor *E. coli* strain to *Aromatoleum evansii* by conjugation, using a method according to the art.

The thus obtained *Aromatoleum evansii* pre-culture containing the plasmid encoding the AorA ebA5004, AorB ebA5005 and AorC ebA5007 subunits and the ampicillin resistance gene were grown on an anaerobic minimal medium with medium nitrates for *Thauera aromatica* species (DSMZ Medium 586, see table 2) with the addition of ampicillin so that the final concentration of the antibiotic is 100 µg/ml and addition of 18 nM Na₂WO₄.

**Table 2. DSMZ Medium 586 ingredients and preparation.**

| | | | |
|---|---|---|---|
| **Solution A:** | **Amount** | Adjust solutions A and B to pH 7.8 for DSM 6898, or to pH 7.2 for other DSMZ strains, | |
| KH₂PO₄ | 0.816 g | | |
| KH₂PO₄ | 5.920 g | autoclave separately and combine after cooling. Add 10 ml of sterile trace elements solution SL-10 (see below) and 5 ml vitamin solution | |
| Distilled water | 500 ml | | |
| **Solution B:** | | | |
| NH₄Cl | 0.530 g | | |
| MgSO₄ × 7 H₂O | 0.2 g | **Trace element solution**: First dissolve FeCl₂ in the HCI, then dilute in water, add and dissolve the other salts. Finally make up to 1000.0 ml | |
| KNO₃ | 2.0 g | | |
| CaCl₂ × 2 H₂O | 0.025 g | | |
| Sodium benzoate | 0.72 g | | |
| Distilled water | 500 ml | **Vitamin solution**: Stir for some hours, filter sterilize the solution. | |
| | | | |
| Vitamin solution | | Trace element solution | **Amount** |
| Vitamin B12 | 50.0 mg | HCI (25%; 7.7 M) | 10.0 ml |
| Pantothenic acid | 50.0 mg | FeCl₂ × 4 H₂O | 1.50 g |
| Riboflavin | 50.0 mg | ZnCl₂ | 70.0 mg |
| Pyridoxamine-HCI | 10.0 mg | MnCl₂ × 4 H₂O | 100.0 mg |
| Biotin | 20.0 mg | H₃BO₃ | 6.0 mg |
| Folic acid | 20.0 mg | CoCl₂ × 6 H₂O | 190.0 mg |
| Nicotinic acid | 25.0 mg | CuCl₂ × 2 H₂O | 2.0 mg |
| Nicotine amide | 25.0 mg | NiCl₂ × 6 H₂O | 24.0 mg |
| α-lipoic acid | 50.0 mg | Na₂MoO₄ × 2 H₂O | 36.0 mg |
| p-aminobenzoic acid | 50.0 mg | | |
| Thiamine-HCI × 2 H₂O | 50.0 mg | | |
| Distilled water | 1000.0 ml | | |

Cultivation was carried out in anaerobic bottles in a non-shaking incubator (30°C, 7 days) with periodic supplementation of nitrates and sodium benzoate (to a concentration of 10 mM and 4 mM, respectively), monitoring nitrate and nitrite levels. The resulting culture was diluted 100-fold in the minimum medium described above, and the cultivation was continued until the OD578 optical density was 0.6. After it was reached, the temperature in the incubator was lowered to 18°C and the enzyme overexpression was induced by the addition of anhydrotetracycline (AHT) to a concentration in the culture medium of 200 ng /ml. After 20 h of culture, cells were separated from the culture medium by centrifugation at 4500 g (1 h, 4°C). The cell suspension was then lysed by sonication (Sonics Vibra-Cell VCX500, intermittent cycle, 5 min, amplitude 40%, energy 150,000 J) or pressure homogenization with a French press, and cell debris was separated by ultracentrifugation at 40,000 g angular acceleration for 1 hour at 4°C. The cell-free extract was then applied to a Strep-tag^{®} II column (5 mL IBA GmbH), and after rinsing with a stock buffer (100 mM tris(hydroxymethyl)aminomethane hydrochloride (Tris-HCI) pH 8.0, 150 mM sodium chloride, 10% (w/v) glycerol), the enzyme was eluted with elution buffer containing stock buffer enriched with 5 mM desthiobiotin. The enzyme thus obtained was frozen at -80°C and stored until used in the reaction.

The procedure carried out in this way yielded from 2 g of wet cell mass 14 ml of enzyme preparation with an activity of 25 nmol mg⁻¹ min⁻¹ in the oxidation of benzyl aldehyde with benzyl viologen as an electron acceptor.

### Examples of the NAD⁺ reduction by the process according to the invention.

### Example 3. Reduction of NAD⁺ by biocatalytic method at pH 8, 30°C, using homogeneous purified AOR protein.

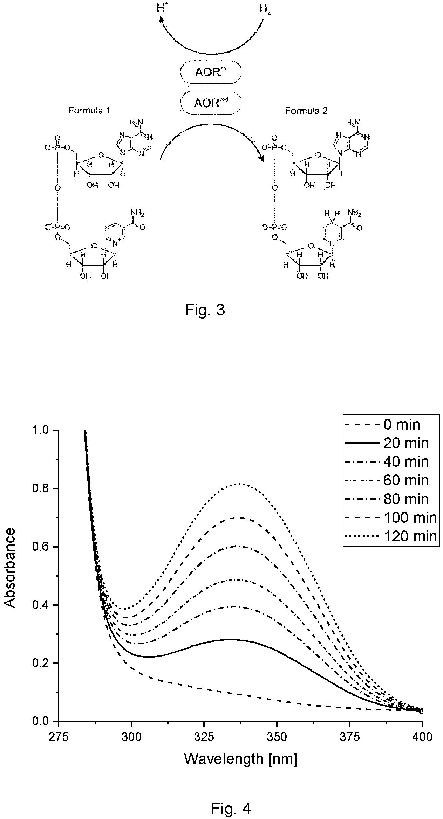

The reaction, which was represented on Fig. 3, was carried out under anaerobic conditions in a 2 ml batch reactor (spectrophotometric cuvette). 1 ml of 50 mM TRIS/HCI buffer pH 8.0 and 0.5 mg of NAD⁺ were introduced into the reactor in an oxygen-free atmosphere with a hydrogen content of 2.5% (v/v). The reaction was initiated by the addition of the purified enzyme preparation with AOR activity (recombinantly expressed in *A. evansii* under anaerobic conditions) in a mass ratio to NAD⁺ 1:25. The reaction was carried out at a temperature of 30°C. The course of the reduction of dinucleotide from the oxidized form (NAD+) to the reduced form (NADH) was followed spectrophotometrically by scanning in the wavelength range of 200 to 400 nm every 20 min and shown in Fig. 4.

### Example 4. Reduction of NAD⁺ by biocatalytic method at pH 6, 15°C, using homogeneous purified AOR protein.

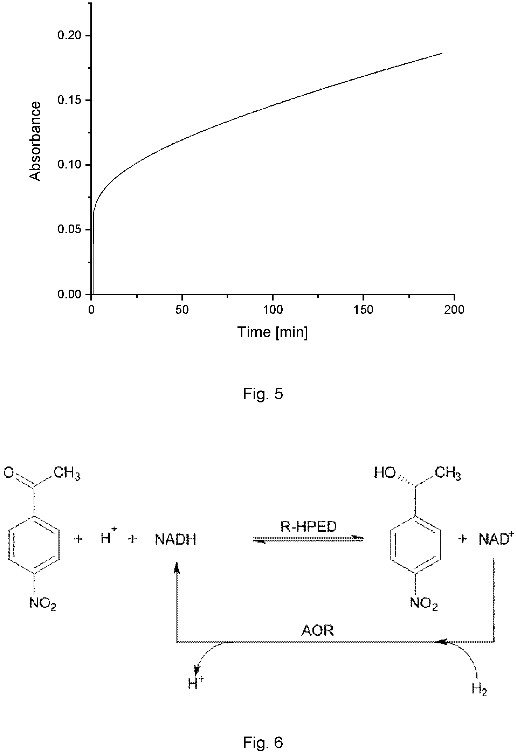

The reaction was carried out under anaerobic conditions in a 1.5 ml batch reactor, adapted to measure the absorbance of the solution (spectrophotometric cuvette). 1 ml of 50 mM MES buffer at pH 6.0 and 0.15 mg of NAD⁺ was introduced into the reactor placed in an oxygen-free atmosphere with a hydrogen content of 2.5% (v/v). The reaction was initiated by the addition of purified enzyme preparation with AOR activity in a weight ratio to NAD⁺ of 1: 3. The reduction was carried out at 15°C. The course of the reduction of dinucleotide from the oxidized form (NAD⁺) to the reduced form (NADH) was followed spectrophotometrically at 340 nm for 190 minutes, as shown in Fig. 5.

### Example 5. Regeneration of NADH with AOR from A. aromaticum and hydrogen for the reaction of 4-nitroacetophenone reduction to (R)-1-(4'-nitrophenyl)ethanol catalysed by the R-HPED from A. aromaticum.

The example demonstrates the practical use of the biocatalytic regeneration of NADH from NAD⁺ with hydrogen and AOR, which enabled the efficient enzymatic synthesis of (R)-1-(4'-nitrophenyl)ethanol from 4'-nitroacetophenone using NADH-dependent alcohol dehydrogenase R-HPED from *A. aromaticum.* Reaction scheme is presented in Fig. 6.

The reaction was carried out in a batch reactor with a volume of 20 ml at a temperature of 30°C. 4.5 ml of 50 mM MES buffer at pH 7.3 and 2 mg of NAD⁺ and the purified enzyme preparation with AOR activity in a mass ratio to NAD⁺ of 1:2 were introduced into the reactor in an oxygen-free atmosphere with a hydrogen content of 3% (v/v). 4-nitroacetophenone was dissolved in acetonitrile in an amount giving 200 mM substrate solution (so-called stock). 50 µl of the 4'-nitroacetophenone stock was added to the reaction yielding the concentration in the reaction mixture of 2 mM. The reaction was initiated by the addition of the enzyme preparation with R-HPED activity in a volume of 5 µl of an aqueous solution with a protein concentration of 2.8 mg/ml to the reaction mixture. The molar ratio of AOR protein to R-HPED protein in this reactor was 6.5:1. In the reactor, 4'-nitroacetophenone was reduced to (R)-1-(4'-nitrophenyl)ethanol with NADH electron donor, which was generated from NAD⁺ by H₂ and AOR. The course of the reaction was monitored by HPLC with DAD detection of the substrate, *i.e.* 4'-nitroacetophenone. After 4 hours, a conversion of 4'-nitroacetophenone of 22% (0.44 mM) was observed.

### Example 6. Regeneration of NADH with crude extract of AOR from recombinant A. evansii and hydrogen for the reaction of acetophenone reduction to (R)-1-phenylethanol catalysed by -HPED from A. aromaticum.

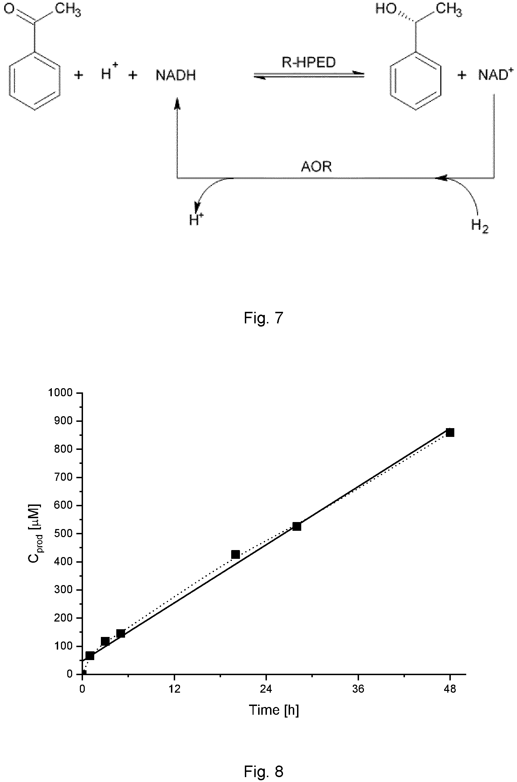

The example demonstrates the practical use of the biocatalytic regeneration of NADH from NAD⁺ with hydrogen and a crude extract of recombinant AOR from *A. evansii,* which enabled the efficient enzymatic synthesis of (*R*)-1-phenylethanol from acetophenone using NADH-dependent alcohol dehydrogenase R-HPED from *A. aromaticum* (Fig. 7).

The crude extract was obtained according to the following procedure. *A. evansii* bacteria, containing plasmid encoding AOR, were cultivated in 1 L bottle under anaerobic conditions according to the procedure provided in example 2. At the end of cultivation, the bacteria were separated from the medium by centrifugation and the pellet was resuspended in 40 ml of lytic buffer (100 mM Tris/HCI pH 8,0, 150 mM NaCl, 10% glycerol (v/v)) containing DNAze I and lysozyme. The suspension was homogenized using the ultrasonic method (Sonics Vibra-Cell VCX500, intermittent cycle, 5 min, amplitude 40%, energy 150,000 J). In the next step, the soluble protein fraction was separated from the membrane fraction by 30-minute ultracentrifugation at 4°C and 4000 rcf. The supernatant was 5-fold concentrated by ultrafiltration on the membrane with 10 kDa cutoff and diluted with the lytic buffer in a 1:1 ratio. The protein concentration in such extract was 30 mg/ml.

The reaction was carried out in a batch reactor with a volume of 20 ml at a temperature of 30°C. 4.5 ml of Tris/HCI o pH 8.0, NAD⁺ (final concentration 0.5 mM) and 0.5 ml of the crude extract of AOR with a stock concentration of 30 mg/ml were introduced into the reactor under the oxygen-free atmosphere with a hydrogen content of 3% (v/v).

Acetophenone was dissolved in acetonitrile in an amount giving 300 mM substrate solution (stock solution). Then 50 µl of the acetophenone stock was added to the reaction yielding the concentration in the reaction mixture of 3 mM. The reaction was initiated by the addition of the enzyme preparation with R-HPED activity in a volume of 10 µl of an aqueous solution with a protein concentration of 2.8 mg/ml to the reaction mixture. In the reactor, acetophenone was reduced to (*R*)-1-phenylethanol with NADH electron donor, which was generated from NAD⁺ with AOR and H₂. The course of the reaction was monitored by HPLC with DAD detection of the product, *i.e.* (*R*)-1-phenylethanol (Fig. 8). The product was formed with an average rate of 18 µM/h which did not decrease during 48 h of reaction. After 48 hours, a conversion of acetophenone of 28.6% (0.86 mM) was observed.

### Example 7. Reduction of NAD⁺ by biocatalytic method at pH 8, 30°C, with a cell extract containing recombinant AOR from A. evansii and hydrogen from a gas mixture containing CO.

The example demonstrates the possibility of using gases of various compositions as a hydrogen source, including carbon monoxide, nitrogen, hydrogen and trace amounts of oxygen (a mixture similar to those produced during biomass gasification) for the biocatalytic regeneration of NADH from NAD⁺ using hydrogen and a cell extract from *A . evansii* with AOR activity.

The crude extract was obtained according to the following procedure. *A. evansii* bacteria, containing plasmid encoding AOR, were cultivated in 1 L bottle under anaerobic conditions according to the procedure provided in example 2. At the end of cultivation, the bacteria were separated from the medium by centrifugation and the pellet was resuspended in 40 ml of lytic buffer (100 mM Tris/HCI pH 8,0, 150 mM NaCl, 10% glycerol (v/v)) containing DNAze I and lysozyme. The suspension was homogenized using the ultrasonic method (Sonics Vibra-Cell VCX500, intermittent cycle, 5 min, amplitude 40%, energy 150,000 J). In the next step, the soluble protein fraction was separated from the membrane fraction by 30-minute ultracentrifugation at 4°C and 4000 rcf. The supernatant was 5-fold concentrated by ultrafiltration on the membrane with 10 kDa cutoff and diluted with the lytic buffer in a 1:1 ratio. The protein concentration in such extract was 30 mg/ml.

The reaction scheme shown in Fig. 2 was carried out under anaerobic conditions in a 2 ml batch reactor (spectrophotometric cuvette). 1 ml of 50 mM TRIS/HCI buffer at pH 8.0 and 50 µl of cell extract with AOR activity were introduced into the reactor. The atmosphere in the reactor was changed by flushing the liquid with 6 ml of the target gas mixture with a gas mixture containing (v/v) 59% nitrogen, 40% carbon monoxide, approx. 1% hydrogen and approx. 0.03% oxygen. The reactor prepared in this way was incubated at 15°C for 1 hour in order to saturate the liquid phase with gases.

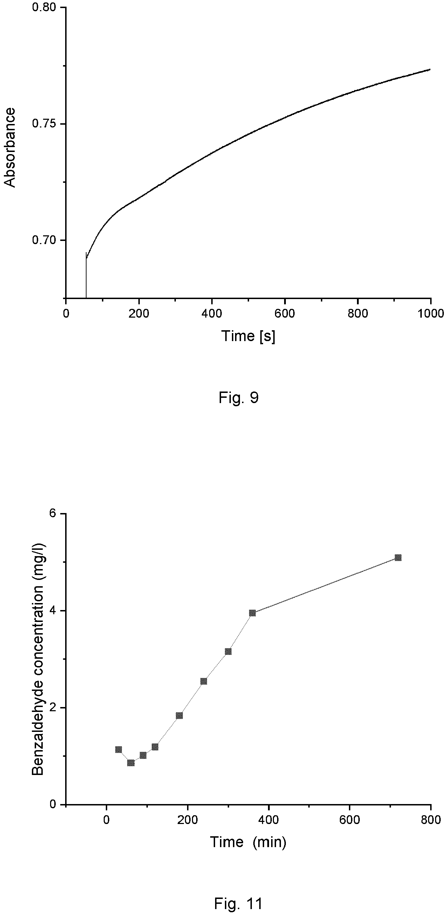

Then the reactor was heated to 30°C, 50 µl of a solution containing 0.6 mg NAD⁺ was added and the course of the reduction of dinucleotide from oxidized (NAD⁺) to reduced form (NADH) was followed spectrophotometrically at 340 nm for 15 minutes, as shown in Fig. 9.

### Examples of the preparation of aliphatic and aromatic aldehydes by the process according to the invention.

### Example 8. Synthesis of benzaldehyde by biocatalytic reduction of benzoic acid using the homogeneous purified AOR protein from A. aromaticum SR7Δpdh

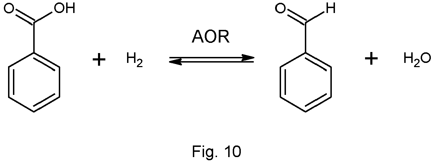

The reaction, which diagram is shown in Fig. 10, was carried out under anaerobic conditions in a 10 ml mixed batch reactor. 3 ml of a 50 mM buffer based on 2-(N-morpholino)ethanesulfonic acid (MES buffer) with a pH of 6.0 is introduced into the mixing vessel placed in an oxygen-free atmosphere at atmospheric pressure (1 atm) with a hydrogen content of 2.5% (v/v), 30 mg of sodium benzoate and 10 µl of a solution containing 5 mg/ml sodium dithionite to remove residual oxygen from the buffer. The reaction is initiated by adding the purified enzyme preparation with AOR activity (obtained in the culture of *A. aromaticum* SR7 *Δpdh* and enzyme isolation under anaerobic conditions) in a weight ratio to acid 1:65. The reaction is carried out at 25°C with stirring (50 rpm). The course of the reaction is monitored by determining the concentration of the product in the analyzed samples by using high-performance liquid chromatography coupled to a triple quadrupole mass spectrometer (LC-MS/MS) in Fragmentation Reaction Monitoring (MRM) mode. After 12 hours of reaction, the benzaldehyde concentration was 5 mg/l. The increase in product concentration over time is shown in Fig. 11.

### Example 9. Synthesis of trans-cinnamaldehyde by biocatalytic reduction of cinnamic acid using homogeneous purified AOR protein from A. aromaticum.

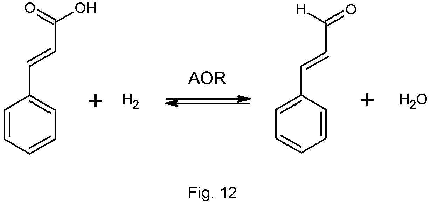

The reaction, which scheme is shown in Fig. 12 was carried out under anaerobic conditions in a 10 ml mixed batch reactor. 3 ml of 50 mM buffer based on 2-(*N*-morpholino)ethanesulfonic acid (MES buffer) pH 6.5 was introduced into the mixing vessel placed in an anaerobic atmosphere at atmospheric pressure (1 atm) with a hydrogen content of 3% (v/v). and 15 mg of trans-cinnamic acid dissolved in 0.5 ml of tert-butanol. The reaction was initiated by adding the purified enzyme preparation with AOR activity from native *A. aromaticum* bacteria in a weight ratio 1:75 to acid. The reaction was carried out at 30°C with stirring (50 rpm). The course of the reaction was monitored by determining the concentration of the product in the analyzed samples by monitoring the fragmentation reaction (MRM) on LC-MS/MS. After 6 hours of the reaction, the concentration of trans-cinnamaldehyde was 3 mg/l.

### Example 10. Synthesis of octanal by biocatalytic reduction of octanoic acid using homogeneous purified recombinant AOR protein from A. evansii and identification of reaction products by GC-MS.

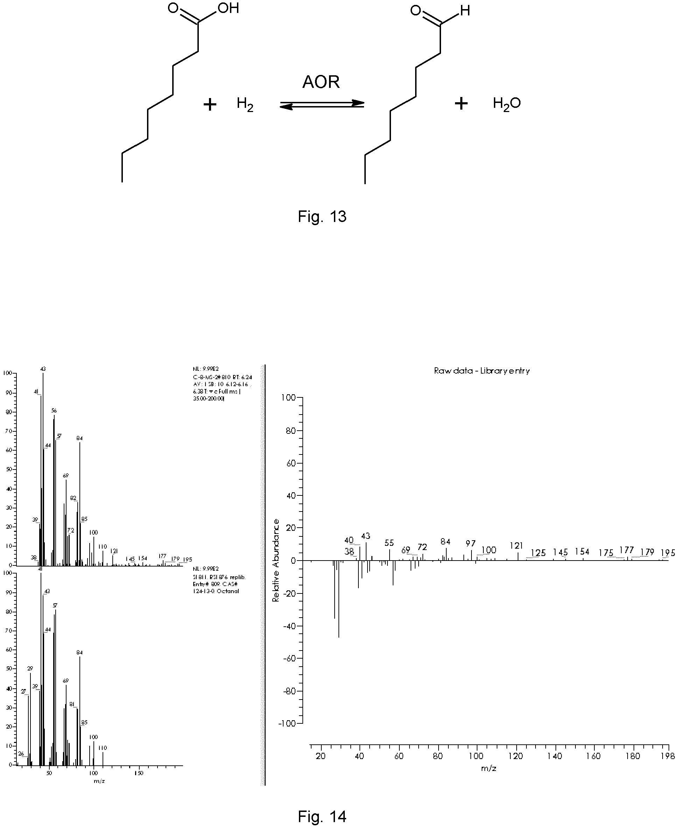

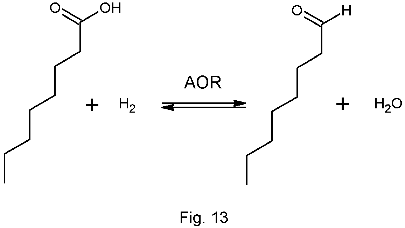

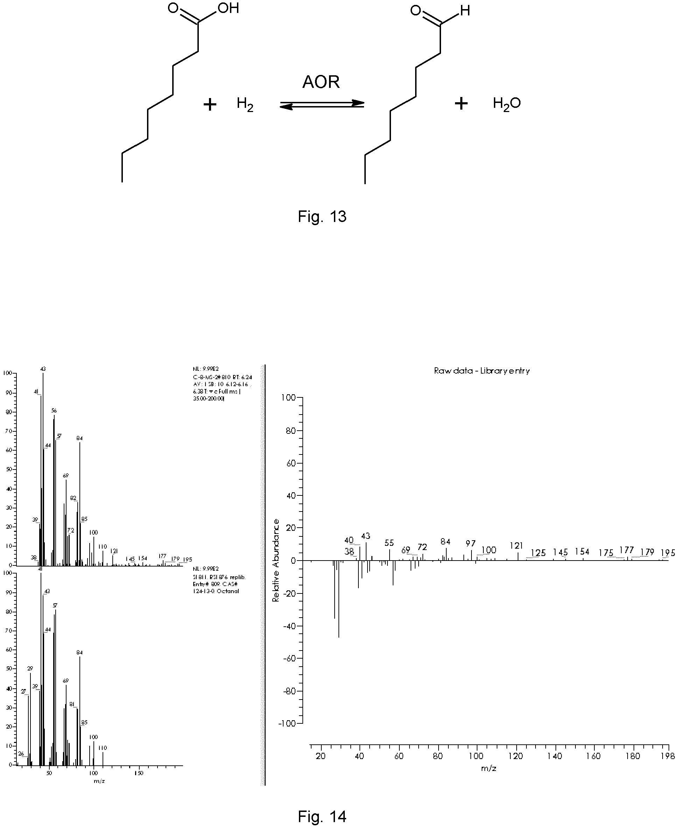

The reaction, which scheme is shown in Fig. 13, was carried out under anaerobic conditions in a stirred tank batch reactor with a volume of 20 ml. 10 ml of 50 mM buffer based on 2-(N-morpholino) ethanesulfonic acid (MES buffer) with pH 6.5 and 30 mg of octanoic acid were introduced into the mixing vessel placed under an anaerobic atmosphere with a hydrogen content of 100% (v/v) and a pressure of 1 atm. The reaction was initiated by adding a purified enzyme preparation with AOR activity derived from overexpression in *A. evansii* in a weight ratio to acid 1:35. The reaction was carried out at 20°C with stirring at 50 rpm. The reaction product was isolated by solid-liquid extraction (SPE) on columns with a sorbent bed (so-called SPE columns).

The extraction procedure was carried out as follows: a hydrophobic SPE bed (e.g. C18 octadecyl bed) was conditioned according to the rules of the chemical art, and then, after rinsing with water, the reaction mixture was applied, the bed was washed with one column volume of water to remove octanoic acid, and then the bed was dried with airflow. The dry column was wetted with methanol and then the adsorbed product was eluted with one column volume of methanol.

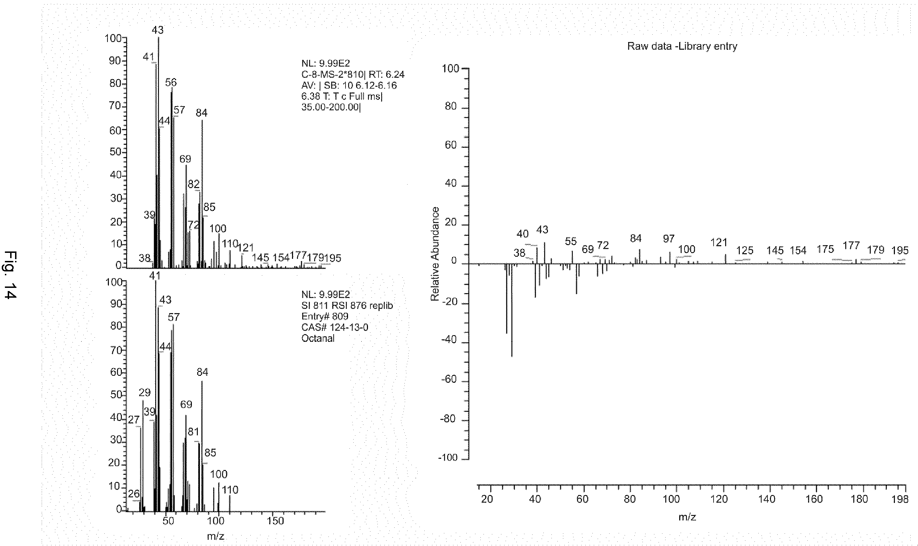

The product was identified in the eluent by the GC-MS method (scanning mode, positive ions, MS-5 column, the temperature gradient in the range of 60-290°C) on the basis of comparison of the MS spectrum of the isolated product with the octanal spectrum in the NIST replib library and with the octanal standard spectrum (Fig. 14).

### Example 11. Synthesis of benzaldehyde by biocatalytic reduction of the benzoic acid using a homogeneous recombinant protein from AOR A. evansii and identification of reaction products by HPLC.

The reaction was carried out with the catalyst in the form of a cell extract obtained from the culture of *Aromatoleum evansii* bacteria containing the anhydrotetracycline-induced plasmid pASG_105_AOR_full. In order to obtain the preparation of the cell extract, the bacterial cells obtained from centrifugation of 3 liters of culture acc. example 2 has been opened in 40 ml lysis buffer (100 mM TRIS buffer pH 8.0, 150 mM NaCl and 10% (v/v) glycerol) supplemented with DNAse I and lysozyme, sonicated at 4°C for 20 minutes ( Sonics Vibra -Cell VCX500, intermittent cycle, 5 min, amplitude 40%, energy 150,000 J). The enzyme preparation was separated from the membrane fraction by centrifugation at 4°C 40 000 rcf for 30 minutes. The supernatant was concentrated 5-fold on a membrane with a particle permeability of less than 10 kDa and diluted 2-fold with lysis buffer.

In the 150 ml reactor, 45 ml of 50 mM buffer based on 2-(*N*-morpholino)ethanesulfonic acid (MES buffer) pH 5.65 and 350 mg of benzoic acid neutralized with 1 ml of sodium hydroxide in the acid: base molar ratio 1:1 (M:M) and 2 ml of cell extract preparation was mixed. The reaction was carried out in a pressure reactor at a pressure of 2 bar of pure hydrogen with the agitation of 100 rpm and at 25°C. The reaction was initiated by replacing the air atmosphere of the reactor with hydrogen. After 4 h and 30 minutes, the reaction was quenched by adding 5 ml of 5 M hydrochloric acid. Acidifying the reaction mixture to precipitate proteins derived from the cell extract and precipitation of the substrate, *i.e.* benzoic acid, which allows the separation of these components from the product by centrifugation at 40 000 rcf. After centrifugation, the supernatant was separated and the pH was adjusted to 9.0 with 20% ammonia water. The reaction product was isolated by solid-liquid extraction on SPE columns. For this purpose, a reaction mixture was applied on a 100 mg SPE bed (for example the Strata X polymer bed produced by Phenomenex) after a conditioning step according to the rules of the art. The column was then washed with one volume of water with 5% (v/v) methanol and 2% (v/v) ammonia water to remove the buffer and residual substrate. The adsorbed product was eluted with one column volume of methanol with 2% (v/v) acetic acid. The product concentration was determined by HPLC. In the above manner, 25 µg of benzaldehyde with the purity of HPLC was synthesized.

### Example 12. Synthesis of 4-hydroxybenzaldehyde by biocatalytic reduction of the 4-hydroxybenzoic acid using a homogeneous protein purified from AOR A.aromaticum and identification of reaction products by MRM LC-MS/MS.

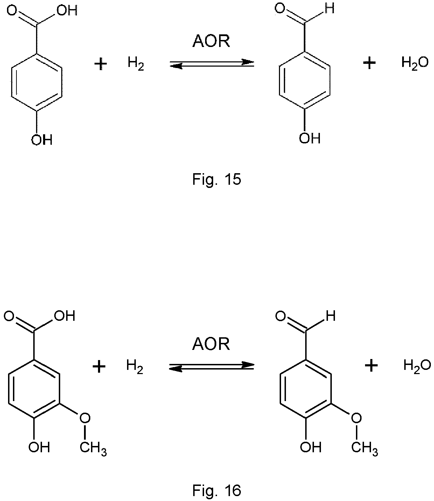

The reaction, which scheme is shown in Fig. 15, was carried out under anaerobic conditions in a 10 ml mixed batch reactor. 4 ml of 50 mM buffer based on 2-(*N*-morpholino)ethanesulfonic acid (MES buffer) pH 6.0 was introduced into the mixing vessel placed in an oxygen-free atmosphere at atmospheric pressure (1 atm) with a hydrogen content of 5% (v/v) and 8 mg of 4-hydroxybenzoic acid neutralized with sodium hydroxide in a 1:1 molar ratio was added. The reaction was initiated by adding the purified enzyme preparation with AOR activity from native *A. aromaticum* bacteria in weight to acid ratio of 1:8. The reaction was carried out at 25°C with stirring (50 rpm). The course of the reaction was monitored by determining the concentration of the product in the analyzed samples by LC-MS/MS in mode monitoring fragmentation reaction (MRM). After 4 hours of the reaction, the concentration of 4-hydroxybenzaldehyde was 0.6 mg/l.

### Example 13. Synthesis of vanillin by biocatalytic reduction of vanillic acid using homogeneous purified recombinant AOR protein from A. evansii and identification of reaction products by GC-MS.

The reaction, which scheme is shown in Fig. 16, was carried out under anaerobic conditions in a stirred tank batch reactor with a volume of 10 ml. 10 ml of 50 mM buffer based on 2-(N-morpholino) ethanesulfonic acid (MES buffer) with pH 5.5, 67.3 mg of vanillic acid and 3 drops of 2 M NaOH were introduced into the mixing vessel placed under an anaerobic atmosphere with a hydrogen content of 2.5% in nitrogen (v/v) and a pressure of 1 atm. The reaction was initiated by adding a purified enzyme preparation with AOR activity derived from overexpression in *A. evansii* in a weight ratio to acid 1:90. The reaction was carried out at 20°C with stirring at 50 rpm. The reaction product was isolated by solid-liquid extraction (SPE) on columns with a sorbent bed (so-called SPE columns).

The extraction procedure was carried out as follows: a hydrophobic SPE bed (e.g. C18 octadecyl bed) was conditioned according to the rules of the chemical art, and then, after rinsing with water, the reaction mixture was applied, the bed was washed with one column volume of water to remove vanillic acid, and then the bed was dried with airflow. The dry column was wetted with methanol and then the adsorbed product was eluted with one column volume of methanol.

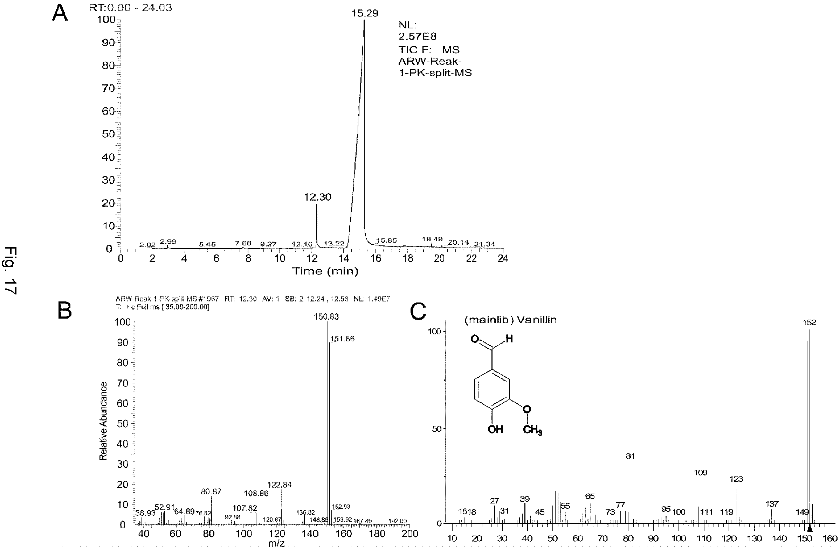

The product was identified in the eluent by the GC-MS method (scanning mode, positive ions, MS-5 column, the temperature gradient in the range of 60-290°C) on the basis of comparison of the MS spectrum of the isolated product with the vanillin spectrum in the NIST mainlib library and with the vanillin standard spectrum (Fig. 17).

### Example 14. Synthesis of nicotinic aldehyde by biocatalytic reduction of the nicotinic acid using a homogeneous protein purified from AOR A.aromaticum and identification of reaction products by GC-MS.

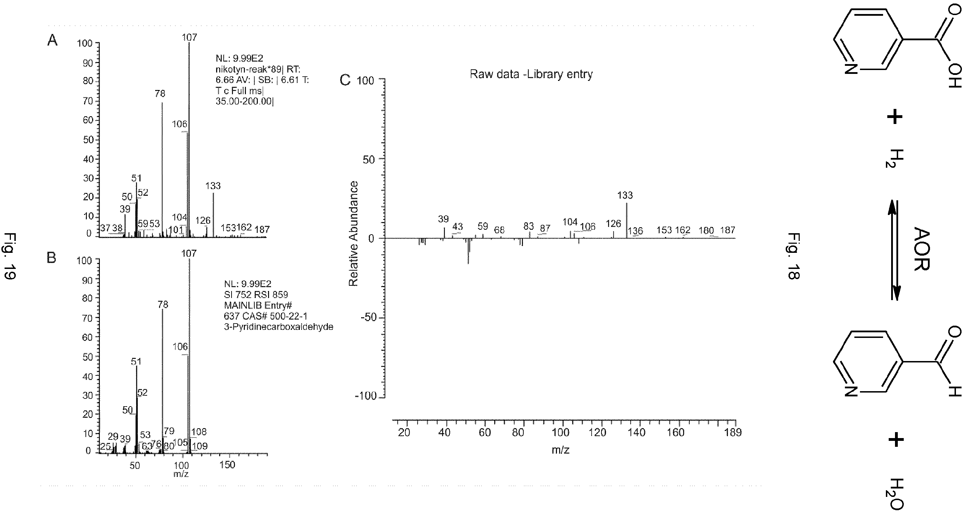

The reaction, which scheme is shown in Fig. 18, was carried out under anaerobic conditions in a 30 ml mixed batch reactor. 20 ml of 50 mM buffer based on 2-(N-morpholino)ethanesulfonic acid (MES buffer) pH 6.0 was introduced into the mixing vessel placed in an oxygen-free atmosphere at atmospheric pressure (1 atm) with a hydrogen content of 5% (v/v) and 100mg of nicotinic acid neutralized with sodium hydroxide in a 1:1 molar ratio was added. The reaction was initiated by adding the purified enzyme preparation with AOR activity from native *A. aromaticum* bacteria in a weight to acid ratio of 1:8. The reaction was carried out at 25°C with stirring (50 rpm) for 6 hours. The reaction was quenched by adding 5 ml of 5 M hydrochloric acid. Acidifying the reaction mixture to precipitate enzymes and precipitation of the substrate, *i.e.* nicotinic acid, which allows the separation of these components from the product by centrifugation at 40 000 rcf. After centrifugation, the supernatant was separated and the pH was adjusted to 9.0 with 20% ammonia water. The reaction product was isolated by solid-liquid extraction on SPE columns.

For this purpose, a reaction mixture was applied to a 100 mg SPE bed (for example the Strata X polymer bed produced by Phenomenex) after a conditioning step according to the rules of the art. The column was then washed with one volume of water with 5% (v/v) methanol and 2% (v/v) ammonia water to remove the buffer and residual substrate. The adsorbed product was eluted with one column volume of methanol with 2% (v/v) acetic acid.

The product was identified in the eluent by the GC-MS method (scanning mode, positive ions, MS-5 column, the temperature gradient in the range of 60-290°C) on the basis of comparison of the MS spectrum of the isolated product with the 3-pyridinecarboxaldehyde spectrum in the NIST replib library (Fig. 19).

## Claims

1. The method of the enzymatic reduction of the NAD⁺ of the general formula 1 to the the reduced nicotine adenine dinucleotide 1,4-NADH of the general formula 2 and carboxylic acids of the general formula 3 to the aldehydes of the general formula 4, 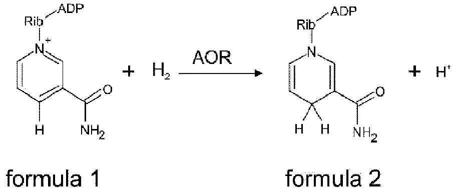 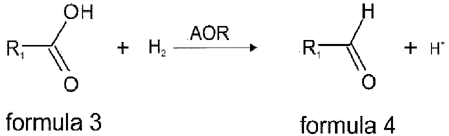 where
Rib is ribose,
ADP is adenosine 5'-diphosphate,
R₁ is an aliphatic chain with a length of one to nine carbon atoms, or an aromatic group, or a furan group, or a thiofuran group, or a 3-pyrimidine group, or a benzyl group, or a propionic group, or ethylcarboxyl group, or a 2-ethylphenyl group or an ethyl-1,2-diol group, or 2-propene group, or C₆H₅ group, or F-C₆H₄ group, or Cl-C₆H₄ group, or Br-C₆H₄ group, or NH₂-C₆H₄ group, or OH-C₆H₄ group, or OMe-C₆H₄ group, or 4-OH-3-OMe-C₆H₃ group;
whereas, the regioselective reduction is conducted under an anaerobic atmosphere containing hydrogen, by an enzyme preparation with tungsten aldehyde oxidoreductase (AOR) activity with an amino acid sequence of at least 70% identity to the sequence deposited in the Universal Protein database Resource Uniprot under the following gene names for subunits AorA ebA5004, AorB ebA5005 and AorC ebA5007, derived from the bacterium *Aromatoleum aromaticum* EbN1, or from the genetically modified bacterium *A. aromaticum* strain SR7*Δpdh*, or from the recombinant overexpression system in *Aromatoleum evansii* containing a plasmid with genes coding at least the AOR enzyme *(i.e.* aorA, aorB, aorC genes) and potentially the gene encoding chaperone proteins (aorD and aorE), whereby the regioselective reduction of the substrate of formula 1 to product of formula 2 is carried out in an aqueous environment, at a temperature of 5 to 40°C, in the reaction mixture, which contains:
- a buffer providing a pH in the range of 4.0-9.0,
- the carboxylic acid solution at a concentration of 0.1 mM to 100 mM,
the enzyme preparation maintaining the biocatalytic activity of tungsten aldehyde oxidoreductase for a period up to 48 h, which enables the process of regioselective reduction of carboxylic acids has the following course: into the batch reactor under anaerobic atmosphere the buffer, substrate dissolved in water or buffer and enzyme are introduced and then the reaction is initiated by the introduction of the inert gas containing hydrogen or pure hydrogen and the course of the reaction is monitored and product is separated by means of liquid - solid phase extraction or liquid-liquid extraction.

2. The method according to claim 1, **wherein** the regioselective reduction is carried out in an atmosphere consisting of a mixture of hydrogen and inert gas, in which the hydrogen concentration is 0.1-100% (v/v).

3. The method according to any one of claims 1-2, **wherein** the regioselective reduction is carried out in an atmosphere consisting of a synthetic gas which is a mixture of carbon monoxide, inert gas, hydrogen and trace amounts of oxygen (<0.05% v/v).

4. The method according to any one of claims 1-3, **wherein** present in enzyme preparation catalytic enzyme AOR of activity of tungsten aldehyde oxidoreductase is heteromeric, containing at least 3 subunits (AorA, AorB and AorC), with amino acid sequence deposited in Universal Protein Resource database (UniProt) under following gene names for the subunits AorA ebA5004, AorB ebA5005 and Aor C ebA5007, is following:
ebA5004: 
MWKSLHIDPAKCTGCLQCEMACSYEHTGVINPSKSRIKVFSFEHEGRKVPYTCTQC
TEAWCLHSCPVDAIRLDLTTGAKMVFEDTCVGCKVCTIACPFGTINYNQDTGKVQKC
DLCEGDPACAKACPTAAITYIDADWTGLARMQAWAAKANTPASAA,
ebA5005: 
MGWNRKVLRVNLAEGTCTPEPLNMQWADEYLGSRGLATKYLVSETDPKVDPLSPD
NKMIMATGPLTGTMASTGGRYTVVTKGPLTGAIACSNSGGFFGAEMKFAGWDMVIF
EGRSPTPVYLFIENERAELRDASYLWGRSCWETEESIRAQHQDPLIRVSSIGRAGEN
QVMFACIVNDLHRAAGRSGVGAVMGSKNLKAVAIRGTKGVSGIRDFPGFVRATSEA
KKVLAGNPVTSEGLPKFGTQVLMNVINEMGALPTRNHRDVQFEDASKISAEAMHEK
RPSDGKPQLVTNAACFGCTIACGRISAIDKTHFTVKNNPKYWGASGGLEYEAAWAL
 
GAANGVGDLEALQYANLLCNEQGMDPISFGATVGAAMELYETGVLTKERIGLDAPF
GSADALAKLAEMTATGEGFGKEIGLGSKRLCEKYGHPELSMSVKGQEFPAYDSRGI
QGMGLAYATSNRGACHLRGYTVASEVLGVPVKTDPHVIEGKAELVKAFQDATAVFD
SAGICVFTSFAWTLADVQPQIAAACDGDWSMDKLATVGERIWNMERQFNNAAGLG
AQDDNLPPRLTSEPAKSGPAKGMVNRLAEMLPEYYGVRGWTPEGTPTPETLSRLG
LS,
ebA5007:

MKHVILGNGPAGVIAAETLRRAAPTDDILLFGSEDAPPYSRMAIPYLLEGNIDESGTW
LRKSPGHFDRLRIHEMRGRAVSLDSERRRILFDDGHFESWDRLLIATGSHPVRPPIP
GIDLPEVQTCWTLEDARAIARFATPGARVLQLGAGFIGCIIMEALAARGVELTVVEMG
DRMVPRMMTPTAGGMIRKWVEDQGVRVVTNAGVSRIDCRASNDAPLDVTLSTGEV
VVADLVIVAAGVAPNIAFLEATPVHVAKGVLVDDRLQTSVPGIFAAGDVAEAPDLFTG
AHLVAAIQPNAADQARVAALNMAGHEARLKGVLAINVLDTLGLISSSFGQWWGEER
ERGGAGVEHVDEAAYRYLSLQFKDDVLIGATSIGLTEHVGALRGLIHGRVRLGEWKE
RLLHSPLQFVDAYIARSQQPMALVR.

5. The method according to any one of claims 1-4, **wherein** present in enzyme preparation catalytic enzyme AOR has an amino acid sequence identity of at least 70% with respect to the amino acid sequences deposited with Uniprot under the following reference numbers: for the subunit AorA ebA5004, AorB ebA5005 and AorC ebA5007.

6. The method according to claim 1, **wherein** the regioselective reduction process is carried out at a temperature of 15 do 40°C, preferably 30 do 40°C.

7. The method according to claim 1, **wherein** the regioselective reduction process is carried out in an atmosphere consisting of a mixture of hydrogen and inert gas or in pure hydrogen under the pressure of 1-5 atm., preferably under the pressure of 1-2 atm.

8. The method according to claim 1, **wherein** during the regioselective reduction of carboxylic acids, the equilibrium of the reaction is shifted in favour of the aldehyde products by selectively removing the aldehyde products from the reaction medium.

9. The method according to claim 8, **wherein** the selective removal of aldehyde products from the reaction medium is carried out by known liquid-liquid extraction methods.

10. The method according to claim 1, **wherein** the pH of the carboxylic acids reduction reaction is maintained in the range of 4.0-7.5, preferably 4.5-7.0, more preferably 5.0-7.0.

11. The method according to claim 10, **wherein** the pH of the reaction medium is controlled by the organic acid substrate.

12. The method according to claim 1, wherein the pH of the NAD⁺ reduction reaction is maintained in the range of 5.0-9.0.

13. The method according to claim 10 or 12, **wherein** the pH of the reaction medium is controlled by a buffer selected from tris(hydroxymethyl)aminomethane hydrochloride, 2-(*N*-morpholino)ethanesulfonic acid, 2,2-bis(hydroxymethyl)-2,2',2"-nitrilotriethanol hydrochloride, 2-[(2-amino-2-oxoethyl)-(carboxymethyl)amino]acetic acid, phosphate buffer, buffer based on citric acid.

14. The method according to claim 1, **wherein** the regioselective reduction of NAD⁺ is carried out at the carbon 4 of the nicotinic ring of the nicotinamide adenine dinucleotide.

15. The method according to claim 1, **wherein** the regioselective reduction of carboxylic acids is monitored by HPLC, or HPLC coupled with MS, or MS/MS.

16. The method according to claim 1, **wherein** the regioselective reduction of NAD⁺ is monitored by a spectrophotometric method (absorbance measurement at 340 nm).

17. The method according to anyone of the claims 1-16, comprising at least one additional step of recovering and/or purifying the product, whereat the product is either 1,4-NADH of formula 2 itself or a fermentation product produced by a microorganism being supplied with 1,4-NADH of formula 2 produced by the method according to any one of preceding claims 1 to 9, the at least one additional step of recovering and/or purifying comprising at least one recovering or purifying method independently chosen from the list comprising the recovering and/or purifying methods filtration, sedimentation, centrifugation, distillation, liquid-liquid extraction, chromatography, HPLC, adsorption to a solid phase combined with desorption from a solid phase, vacuum-drying, lyophilisation, crystallisation.
